# EUROPEAN PATENT APPLICATION

(11) **EP 4 732 898 A1**
(43) Date of publication of application: **29.04.2026**
(21) Application number: 25210150.6
(22) Date of filing: 21.10.2025
(51) Int. Cl.: A61N 5/10, A61G 13/00

(54) **RADIOTHERAPY COUCH TOP EXTENSION**

(30) Priority: 25.10.2024 GB 202415779
(71) Applicant: Elekta Limited, Crawley, Sussex RH10 9RR (GB)
(72) Inventor: ALEXIS, Henrik, Crawley (GB)
(74) Representative: Thomas, Tomos Daniel

(57) **Abstract**

Disclosed herein is an extension for increasing a width of at least part of a radiotherapy couch top, the extension comprising: a planar portion configured to support part of a subject thereon; and a fixation mechanism configured to fix the extension onto the couch top such that the planar portion overlays at least part of the couch top.

## Description

This disclosure relates to an extension for a radiotherapy couch top, and in particular to an extension for increasing a width of at least part of a radiotherapy couch top. This disclosure also relates to a patient support apparatus and a radiotherapy device including the extension and the radiotherapy couch top.

### Background

Radiotherapy can be described as the use of ionising radiation, such as X-rays, to treat a human or animal body. Radiotherapy is commonly used to treat cancer, for example to treat tumours within the body of a patient or subject. In such treatments, ionising radiation is used to irradiate, and thus destroy or damage, cells which form part of the tumour.

Tumours can be present in various different anatomical locations within a subject. Treatment plans can be generated and optimised to define how particular subjects with particular tumours will be treated, within the confines of what is possible using the available radiotherapy device. Typically, a the subject is disposed on a patient support apparatus and a source of radiation coupled to a rotatable gantry directs a beam of radiation through the subject from various different angles in order to build up the applied dose at the location of the tumour and limit the applied dose at other anatomical locations within the subject. The subject can be positioned on the patient support apparatus in various positions or poses, including with them lying either on their back or front, with either their head or feet closer to the source of radiation, with their arms either above their head or by their sides, etc. The different angles of irradiation and the different poses of the subject, among other things, provide degrees of freedom which can be utilised to optimise irradiation of the tumour and limit irradiation of other tissue.

A patient support apparatus can comprise a support base and a couch top which is supported by the support base and which the subject is disposed on. The couch top is generally a rectangular planar structure taking the form of a board that the subject lies on. If it is desired to accommodate different sizes and/or different poses of subjects, extensions are sometimes provided which can couple to a longitudinal end of the couch top in order to make the couch top longer.

Imaging can be performed to determine a position of a subject and of anatomical features within the subject. Such imaging can be performed before radiotherapy treatment begins with the subject in a treatment position, and/or can be performed while radiotherapy treatment is occurring. This helps ensure that the radiation is directed at the intended location, i.e. the location of the tumour, and not directed at other locations, e.g. locations of organs at risk. Radiotherapy treatment can be paused or adjusted if it is determined that the tumour is receiving or will receive a lower dose than would be desirable and/or if it is determined that healthy tissue is receiving or will receive a higher dose than would be desirable. By way of example, kV imaging (or MV imaging) can be used to provide images for these purposes.

It is desirable for the images produced by the kV imaging to be of high quality/high accuracy and to have as few artefacts as possible. It is also desirable for such images to accurately cover all relevant parts of the subject within a region of interest. This helps ensure that determinations made regarding locations of anatomical features of the subject, and the doses these anatomical features receive, are as accurate as possible. However, a subject is typically disposed on the couch top during such imaging and during such radiotherapy treatment. In order to image the subject, the imaging beam typically passes through the couch top as well as through the subject. Properties of the couch top can therefore introduce unwanted features into the images or otherwise distort the images, which limits the accuracy with which the subject themselves can be imaged.

In addition, the treatment beam also typically passes through the couch top, which attenuates the dose received by the subject. Efforts can be made to take account of this attenuation in treatment plans, for example through increasing the dose applied from a specific angle if it is known that some of this dose will be attenuated due to the treatment beam passing through the couch top. However, there is a risk of small positioning errors being the difference between unattenuated irradiation of the subject and attenuated irradiation of the subject through the couch top, which could lead to dosimetric errors in the irradiation of the subject.

In view of the above, it would be desirable to increase the accuracy of imaging supporting radiotherapy treatment, as well as to increase the accuracy of such radiotherapy treatment itself. It would also be desirable to achieve this increase in accuracy while increasing the versatility of a patient support apparatus to facilitate a greater range of radiotherapy treatments.

The present invention seeks to address these and other disadvantages encountered in the prior art.

### Summary

An invention is defined in the independent claims. Optional features are set out in the dependent claims.

### Figures

Specific embodiments are now described, by way of example only, with reference to the drawings, in which:
Figure 1 depicts a radiotherapy device or apparatus according to the present disclosure;
Figure 2 depicts a patient support apparatus according to the present disclosure;
Figure 3 depicts top-down view of a couch top according to the present disclosure;
Figure 4 depicts an extension according to the present disclosure;
Figures 5a and 5b depict a couch top and extension according to the present disclosure;
Figures 6a and 6b depict an example of fixation of an extension to a couch top according to the present disclosure;
Figure 7 depicts a further example of fixation of an extension to a couch top according to the present disclosure;
Figures 8a and 8b depict a further example of fixation of an extension to a couch top according to the present disclosure.

### Detailed Description

In overview, and without limitation, the application relates to an extension for coupling to a radiotherapy couch top so as to increase a width of at least part of the radiotherapy couch top. The extension includes a planar portion for supporting part of a subject thereon. The extension further includes a fixation mechanism configured to fix the extension onto the couch top such that the planar portion overlays at least part of the couch top, i.e. overlaps with the couch top along a vertical axis. At least part of the lower surface of the extension may be held in contact with at least part of the upper surface of the couch top by the fixation mechanism. The extension, when fixed to the couch top, may extend the areal extent of the couch top in a plan/top-down view. The planar portion can extend the lateral width of the 'head-end' of the couch top such that it can support the legs or arms of the subject. This may enable the combination of the couch top and the extension to support a greater range of poses of the subject.

The shape of the couch top may be optimised to support a torso of the subject on a first, wider, longitudinal portion thereof, and to support a head of the subject on a second, narrower, longitudinal portion thereof. The narrower second portion can be used to support a head of a subject without large amounts of couch top material extending either side of the head of the subject. This enables a treatment beam to have direct access to the head of the subject, without also passing through the couch top, from a greater range of angles, which would otherwise attenuate the treatment beam. The implementation of the 'omega-shape' of the couch top may prevent limitations to reachable treatment zones that may otherwise apply when using couch kicks/couch rotations, and avoid corners of a rectangular couch top colliding with imaging arms or the treatment head. This is of particular importance for brain treatments since it is particularly important to be accurate for these treatments. For such treatments, it is desirable that the narrower longitudinal portion is integrally formed with the wider longitudinal portion, instead of the narrower longitudinal portion itself comprising an extension. This is because it is particularly important for such treatments to avoid the presence of a junction or discontinuity in the treatment/imaging zone, which could otherwise distort or attenuate the treatment/imaging beams. This could reduce the accuracy and reliability of radiotherapy treatment or reduce the treatment regions that could be accommodated.

Instead, the current disclosure provides a couch top which is shaped to generally correspond to the wider torso and narrower head portions of a subject, and an extension which fills in/squares off this shape to make the combination of the couch top and the extension generally rectangular. This is the opposite approach to that typically used in the art for providing couch top extensions. When fixed to the couch top, the extension can support, for example, the legs of the subject in feet-first treatments or the arms of the subject in treatments in which they are disposed on their front with their arms above their heads. The extension can also accommodate the use of treatments using patient positioning devices fixed to the extension and those incorporating immobilisation devices such as vacuum bags fitted over the extension.

Moreover, the current disclosure provides that the extension is coupled to the couch top in a manner which can minimise interference with treatment and imaging beams, while ensuring reliable support of the extension. The extension overlays at least part of the couch top such that it can be generally supported from below by the couch top itself. In addition, a fixation mechanism is provided for fixing the extension onto the couch top therebelow and preventing the extension from flipping over when a load is applied to a lateral edge of the extension (which may not be supported from below by the couch top). This combination of features can avoid the need for implementing fixation mechanisms inside the treatment or imaging zones, which could distort or otherwise reduce the accuracy and reliability of treatment and imaging through such fixation mechanisms.

In addition, the implementation of the extension as described herein may provide gradual changes in the thickness of material present when moving from the couch top to the extension itself. Reducing the amount of material in this region can reduce the risk of applying the wrong dose to the subject.

Accordingly, disclosed herein is an extension for increasing a width of at least part of a radiotherapy couch top, the extension comprising: a planar portion configured to support part of a subject thereon; and a fixation mechanism configured to fix the extension onto the couch top such that the planar portion overlays at least part of the couch top.

As discussed herein, these features enable delivery of more accurate and more reliable radiotherapy treatment for a greater range of poses of the subject. This enables more accurate and more reliable treatment of a greater range of tumors in different anatomical locations and with different sizes/shapes, since radiotherapy treatments can be designed to map onto the extent of particular tumours in more versatile ways. The increased versatility the extension provides increases the range of radiotherapy treatments which can be accurately and reliably delivered.

Optionally, the fixation mechanism is on a lower surface of the planar portion. Optionally, the fixation mechanism comprises a linear protrusion for receiving into and rotating with respect to a linear slot of the radiotherapy couch top. Optionally, the fixation mechanism comprises a pin for receiving into a hole of the radiotherapy couch top. Optionally, the fixation mechanism comprises a rotatable hook for coupling to a pin of the radiotherapy couch top. Optionally, the fixation mechanism comprises a U-shaped edge configured to hook over an edge of the radiotherapy couch top.

Optionally, the planar portion comprises a first longitudinal end and a second longitudinal end, wherein at least a first part of the fixation mechanism is disposed adjacent to the second longitudinal end of the planar portion. Optionally, the extension comprises a cutout at the first longitudinal end thereof configured to receive a positioning accessory. Optionally, the first longitudinal end of the planar portion comprises a chamfered edge. Optionally, the planar portion comprises a first lateral edge and a second lateral edge, wherein at least a second part of the fixation mechanism is disposed adjacent to the first lateral edge and/or the second lateral edge. Optionally, no part of the fixation mechanism is disposed in a laterally central region of the planar portion adjacent to the first longitudinal end of the planar portion.

Optionally, the extension is configured to be selectively connectable to and separable from the radiotherapy couch top.

Also disclosed herein is a patient support apparatus comprising: a radiotherapy couch top; and the extension of any preceding claim for fixing to the radiotherapy couch top using the fixation mechanism.

Optionally, the radiotherapy couch top comprises: a first longitudinal portion with a first lateral width; and a second longitudinal portion with a second lateral width, the second lateral width being smaller than the first lateral width. Optionally, the fixation mechanism is configured to fix the extension to the second longitudinal portion of the radiotherapy couch top such that it overlays the second longitudinal portion of the radiotherapy couch top. Optionally, a lateral width of the extension is approximately equal to the first longitudinal width of the first longitudinal portion of the radiotherapy couch top. Optionally, the combination of the radiotherapy couch top and the extension when fixed together are generally rectangular in plan view. Optionally, the radiotherapy couch top comprises a second fixation mechanism configured to couple to the fixation mechanism of the extension.

Optionally, the first longitudinal portion of the radiotherapy couch top is configured to support a torso of the subject. Optionally, the second longitudinal portion of the radiotherapy couch top is configured to support a head of the subject. Optionally, the extension is configured to support legs of the subject during feet-first radiotherapy treatments.

Optionally, the first longitudinal portion of the radiotherapy couch top is integrally formed with the second longitudinal portion of the radiotherapy couch top. Optionally, the couch top comprises a third longitudinal portion of the radiotherapy couch top longitudinally between the first longitudinal portion and the second longitudinal portion, wherein the first longitudinal portion, the second longitudinal portion and the third longitudinal portion are all integrally formed with each other. Optionally, the third longitudinal portion has a third lateral width which decreases from being equal to the first lateral width at an end of the third longitudinal portion which is adjacent to the first portion, to being equal to the second lateral width at an end of the third longitudinal portion which is adjacent to the second longitudinal portion, and wherein the extension is configured to overlay the second longitudinal portion and the third longitudinal portion.

Also disclosed herein is a radiotherapy device comprising: a rotatable gantry; a radiation source; an imaging apparatus; and the patient support apparatus as described above.

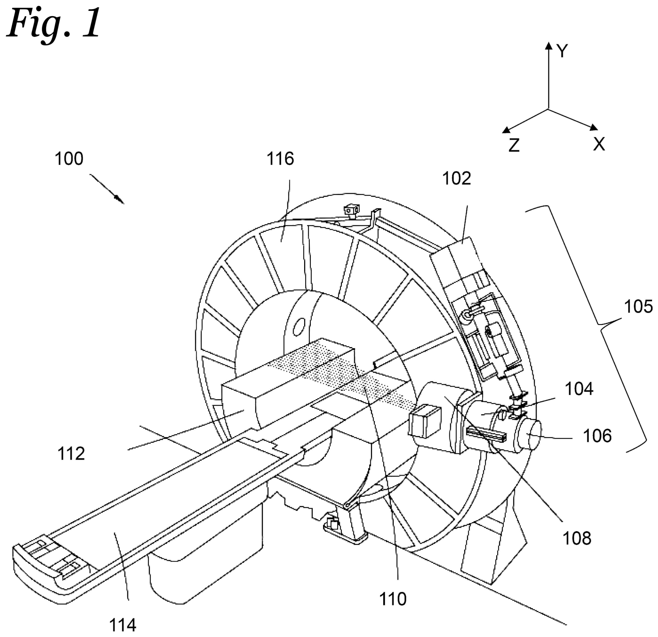

Figure 1 depicts a radiotherapy device 100. The radiotherapy device 100 is suitable for delivering, and configured to deliver, a beam of radiation 110 to a patient during radiotherapy treatment. The device 100 and its constituent components will be described generally for the purpose of providing useful accompanying information for the present application. The device 100 depicted in Figure 1 is in accordance with the present disclosure and is suitable for use with the disclosed systems and apparatuses. While the device 100 in Figure 1 is an MR-linac, in implementations of the present disclosure the device 100 may be another type of radiotherapy device, for example a linac device with a different imaging capability.

In overview, the radiotherapy device 100 comprises a source of radiation 105 and an imaging apparatus 112. The source of radiation 105 is coupled to a rotatable gantry 116. The device 100 comprises a patient positioning apparatus which comprises a patient positioning surface 114 on which a patient may be positioned. Before treatment, the patient is positioned on the surface 114, and the patient positioning apparatus may be used to position the patient in an appropriate position for the treatment, for example according to a reference image on which the patient's treatment plan is based. During treatment, the source of radiation 105 delivers radiation to the patient according to the patient's treatment plan.

The source of radiation 105 is configured to generate a beam of radiation 110, and in particular a beam of therapeutic radiation. The radiation source 105 is attached to the rotatable gantry 116 so as to rotate with the gantry 116. In this way, the radiation source is rotatable around the patient so that the beam 110 can be applied from different angles around the gantry 116. The source of radiation 105 may comprise a beam generation system comprising a linear accelerator (linac). For such a linac device, the beam generation system may comprise a source of RF energy 102, an electron gun 106, and a waveguide 104.

The source 102 of radiofrequency waves, such as a magnetron, is configured to produce radiofrequency waves. The source 102 of radiofrequency waves is coupled to the waveguide 104, for example via a circulator, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source 106 and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide 104 typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun 106. Electrons can be accelerated to speeds approaching the speed of light in the evacuated waveguide 104.

Once the electrons have been accelerated, they travel toward a heavy metal target which, when impacted by the electrons, generates a beam of high energy photons, forming a radiation beam 110. When the electrons strike the target, X-rays are produced in a variety of directions. The device 100 comprises collimation apparatus 108. The collimation apparatus 108 may comprise a primary collimator. The primary collimator is configured to block X-rays travelling in certain directions and pass only forward travelling X-rays to produce a treatment beam 110. The X-rays may be filtered and may pass through one or more ion chambers for dose measuring. The collimation apparatus 108 may additionally comprise beam shaping apparatus such as a multi-leaf collimator (MLC). The beam can be shaped in various ways by the beam-shaping apparatus. The source of radiation is configured to direct the beam 110 of therapeutic radiation, having been appropriately filtered and shaped by the collimation apparatus 108, toward a patient positioned on the patient support surface 114.

The device 100 comprises an imaging apparatus 112 or 'image acquisition apparatus'. The depicted imaging apparatus 112 is an MR imaging apparatus, though the imaging apparatus may take other forms, for example a cone beam computed tomography (CBCT) apparatus. The MR imaging apparatus 112 is shown in cross-section in the diagram. The imaging apparatus 112 is configured to generate imaging data. The imaging data may comprise images of the patient. The imaging apparatus 112 is therefore configured to obtain images of a patient positioned on the patient support surface 114. The imaging data generated by the imaging apparatus 112 may be used to generate a reference image to enable treatment planning, and/or may be used during the delivery of therapeutic radiation to help guide the beam of radiation 110 or to provide an input into motion management and real-time adaptive radiotherapy techniques.

As described herein, the radiotherapy device 100 may comprise an imaging apparatus 112 which is a kV imaging apparatus. While the skilled person will be familiar with kV imaging apparatuses, the following brief description is provided. The kV imaging apparatus may comprise a kV beam source and a kV detector, each of which may be fixed to the rotatable gantry opposite (at 180° to) each other. Each of the kV beam source and the kV detector may be offset by 90° relative to the source of radiation 105. The kV beam source may be configured to generate a kV X-ray beam directed through the subject (and in general through the patient support surface 114), to be incident on the kV detector. The kV detector may be configured to generate an image of the subject based on the kV X-ray beam it receives, as attenuated by the subject (and in general the patient support surface 114) which the kV X-ray beam passes through.

Figure 1 and its accompanying description herein is provided to give context to the application and to facilitate understanding of the present invention. In addition to the components described in overview above, a radiotherapy device also comprises many other functions, components and subsystems as will be understood by the skilled person.

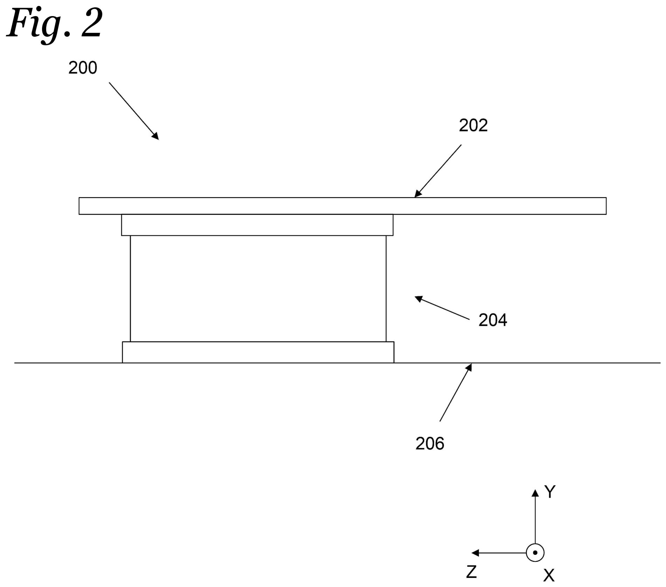

Figure 2 depicts a schematic of a patient support apparatus 200 according to the present disclosure.

The patient support apparatus 200 comprises a couch top 202, i.e. a radiotherapy couch top 202. The patient support apparatus 200, or the couch top 202, may correspond to the patient support apparatus 114 described in relation to Figure 1. The couch top 202 is configured to support a subject, i.e. a patient, thereon. In other words, in use, a subject may be disposed on and in contact with an upper surface of the couch top 202. The couch top 202 may be stiff/rigid enough such that it does not bend or sag significantly when a subject is disposed thereon. The couch top may comprise a foam core with a fibre skin. The fibre skin can comprise, by way of example, carbon fibres or aramid fibres. Such fibres may exhibit adequate strength for supporting the subject while having low atomic numbers, which limits the effect of the fibres on image quality when a kV imaging beam passes through the couch top 202. While the couch top 202 is depicted schematically as being generally flat/linear in the side-on view of Figure 2 for ease of illustration, the present disclosure provides that a couch top 202 has first and second longitudinal portions with first and second lateral widths respectively.

The patient support apparatus 200 also comprises a patient support base 204. The patient support base 204 is configured to support the couch top 202. The patient support base 204 may be disposed on a floor 206 of a room housing the patient support apparatus 200/radiotherapy device 100. The patient support base 204 may comprise one or more drivers, actuators and/or other mechanical/electrical components configured to cause movement of the couch top 202. This movement may comprise linear movement along three perpendicular axes, as well as rotational movement around each of these three perpendicular axes. In other words, the patient support apparatus 200 may be configured for 6D movement, though in some examples the patient support apparatus 200 may not be configured to provide movement along/around one or more of these directions, e.g. may be configured for 3D, 4D or 5D movement.

The couch top 202 may be fixed to the patient support base 204 such that tilting or translation of all or part of the patient support base 204 causes tilting or translation of the couch top 202. This may be used to position the couch top 202, and the subject thereon, into a desired position for imaging and/or radiotherapy. The couch top 202 may be slidably coupled to the patient support base 204, enabling linear movement of the couch top 202 relative to the patient support base 204 (e.g. along the Z-axis as depicted in Figure 2). In some examples, some of the driving electronics or mechanical features may be couped to or integrated into the couch top 202, in a region of the couch top 202 (an 'attachment zone') which is not irradiated by treatment or imaging beams. One or more attachment blocks may be provided (in the attachment zone) between the couch top 202 and the patient support base 204. For example, four such attachment blocks may be provided. The one or more attachment blocks may be configured to couple the couch top 202 to the patient support base 204.

The patient support base 204 may at least be configured to cause the couch top to translate along a left-right direction as depicted in Figure 2 (parallel to the Z-axis). Other components of the radiotherapy apparatus 100 of Figure 1, such as the source of radiation 105 and the imaging apparatus 112, may generally be located to the right of the patient support apparatus 200 as depicted in Figure 2. In a 'setup position' which enables a subject to easily mount the couch top 202, the couch top 202 may be translated/disposed towards the left of Figure 2. The couch top 202 may be translated/disposed to the right as depicted in Figure 2, i.e. towards the other components of the radiotherapy apparatus 100, to place the subject in a 'treatment position' in which they can be imaged and/or treated. The couch top 202 may for example be translated in this manner using a linear guide or linear rail of the patient support base 204 coupled to the couch top 202.

The present application focuses on the form of the couch top 202 as described herein. It will be appreciated that this couch top 202 is generally applicable to various different forms of patient support base 204 and patient support apparatus 200 without limitation, and that the depiction and discussion of Figure 2 is provided merely as an example for understanding the context of the current application. The couch top 202 is the part of the patient support apparatus 200 disposed to contact the subject and configured for support of the subject, in contrast to the parts of the patient support apparatus 200 configured to control/drive movement of the couch top 202 with the subject thereon.

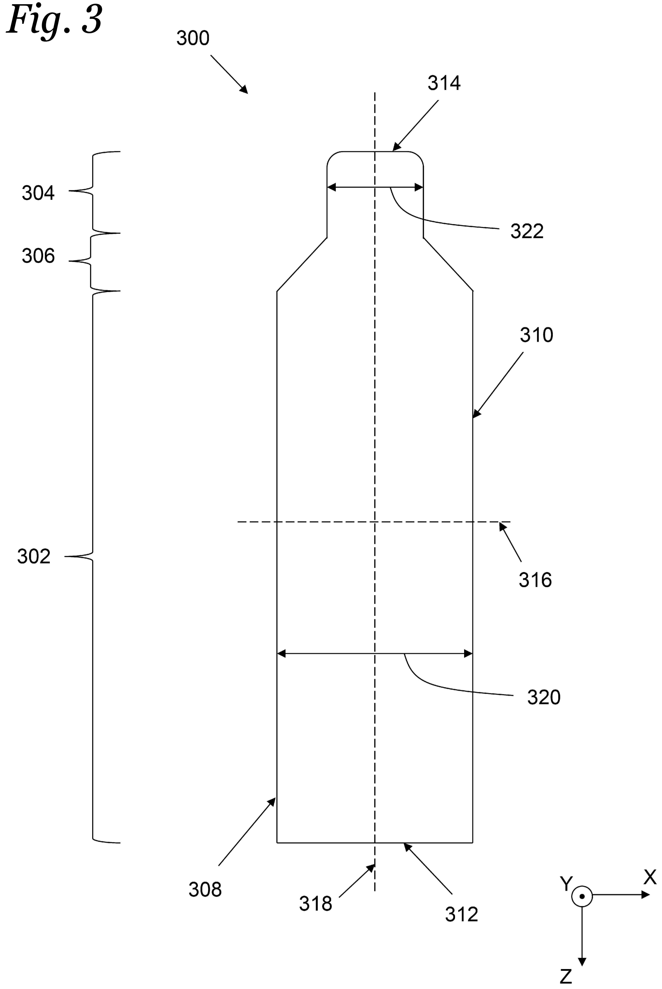

Figure 3 depicts a top-down view of a couch top 300 according to the present disclosure. The couch top 300 may correspond to the couch top 202 described in relation to Figure 2.

The couch top 300 comprises a first longitudinal portion 302 and a second longitudinal portion 304. In other words, the couch top 300 comprises a first portion in a first longitudinal region 302 of the couch top 300 and a second portion in a second longitudinal region 304 of the couch top 300. The couch top 300 may also comprise a third longitudinal portion 306, i.e. a third portion in a third longitudinal region 306 of the couch top, between the first longitudinal portion 302 and the second longitudinal portion 304.

The couch top 300 may comprise a first lateral side 308 and a second lateral side 310. The couch top 300 may comprise a first longitudinal end 312 and a second longitudinal end 314. The couch top 300 may extend between the first lateral side 308 and the second lateral side 310, each of which may be described as being disposed at different ends of a lateral axis 316 of the couch top 300. The couch top 300 may extend between the first longitudinal end 312 and the second longitudinal end 314, each of which may be described as being disposed at different ends of a longitudinal axis 318 of the couch top 300. The longitudinal axis 318 may be perpendicular to the lateral axis 306. The first and second lateral sides 308, 310 may be parallel to the longitudinal axis 318. The first and second longitudinal ends 312, 314 may be parallel to the lateral axis 316.

The first longitudinal portion 302 of the couch top 300 has a first lateral width 320. This lateral width is a width of the couch top 300 along the lateral axis 316 of the couch top 300 between the first lateral side 308 and the second lateral side 310. The couch top 300 may have the first lateral width 320 throughout or in most of the first longitudinal portion 302. The second longitudinal portion 304 of the couch top 300 has a second lateral width 322. This lateral width is a width of the couch top 300 along the lateral axis 316 of the couch top 300 between the first lateral side 308 and the second lateral side 310. The couch top 300 may have the second lateral width 322 throughout or in most of the second longitudinal portion 304.

The second lateral width 322 of the second longitudinal portion 304 is smaller than the first lateral width 320 of the first longitudinal portion 302. In other words, the first longitudinal portion 302 is wider than the second longitudinal portion 304. The second longitudinal portion 304 is narrower than the first longitudinal portion 302. The couch top 300 is not of uniform width along its length (along the longitudinal axis 318 thereof), but instead has a wider portion along a first part of its length and a narrower portion along a second part of its length.

The first longitudinal portion 302 is at or adjacent to the first longitudinal end 312. The first longitudinal portion 302 is disposed closer to the first longitudinal end 312 than the second longitudinal portion 304 is. The first longitudinal portion 302 may comprise an attachment zone configured to couple the couch top 300 to the patient support base 204 of the patient support apparatus 200 (see Figure 2). The attachment zone may be disposed adjacent to the first longitudinal end 312, i.e. at the bottom of Figure 3. The first longitudinal portion 302 may comprise a body treatment zone configured to support a torso of a subject disposed on the couch top 300. The body treatment zone may be disposed closer to the second longitudinal portion 304 than the attachment zone is. The body treatment zone may be disposed between the attachment zone and the second longitudinal portion 304 or between the attachment zone and the third longitudinal portion 306. In use, the couch top 300 may be positioned in relation to other components of the radiotherapy device 100 such that imaging and/or therapeutic radiation passes through the body treatment zone but not through the attachment zone.

The second longitudinal portion 304 is at or adjacent to the second longitudinal end 314. The second longitudinal portion 304 may be comprise or correspond to a head treatment zone configured to support a head of a subject disposed on the couch top 300. In use, imaging and/or therapeutic radiation passes through the head treatment zone. In view of the relative sizes of human torsos and heads, the second longitudinal portion 304 may be shorter (along the longitudinal axis 318) than the first longitudinal portion 302 and is narrower (along the lateral axis 316) than the first longitudinal portion 302.

Since the second longitudinal portion 304 has the narrower second lateral width 322, the head of the subject is more exposed in that a radiotherapy beam can be directed through the head of the subject, without also having to pass through the couch top 300, from a greater range of positions/angles of the source of radiation 105. This may increase the accuracy, efficiency and/or versatility of head treatments. Typically, radiotherapy is applied with the source of radiation 105 located at various angles around the subject using the rotatable gantry 116. It will be appreciated that, if the couch top 300 had the same, first longitudinal width along its whole length, the radiotherapy beam would have to pass through the couch top 300 for irradiation with the source of radiation 105 positioned in at least the lower 180° of the circle described by the rotatable gantry 116. However, since the second longitudinal region 304 is provided with the smaller, second lateral width 322, depending on the particular anatomical target location within the head, this enables direct irradiation of the target without the radiotherapy beam also passing through the couch top 300 with the source of radiation 105 at a greater range of angles. For example, this may be enabled with the source of radiation 105 substantially to the left or right of the couch top 300 (along the X-axis), but below the height of the couch top 300 to some extent (along the Y-axis). This increases the scope for generating treatment plans and corresponding treatments which more accurately target particular locations and shapes of tumours.

The above discussion has focused on the subject being reclined with their head disposed in the second longitudinal portion 304 and their torso disposed in the first longitudinal portion 302. The subject may be disposed on their back with their back in contact with an upper surface of the couch top 300. Alternatively, the subject may be disposed on their front with their front in contact with the upper surface of the couch top 300. Moreover, in some examples the subject may be disposed on the couch top in other ways. For example, in 'feet first' treatments, the subject may be disposed with their head adjacent to the first longitudinal end 312 and their feet adjacent to the second longitudinal end 314. As described herein, an extension or cover board configured to couple to the second longitudinal portion 304 of the couch top 300 may be provided for enabling treatment of the subject in a greater range of poses and thereby providing increased versatility for accurately treating different tumour locations and shapes. The extension may better support the feet/legs of the subject in feet first treatments and may be used for better supporting the arms of the subject in some treatments in which the subject is lying on their front.

The third longitudinal portion 306 may couple the first longitudinal portion 302 to the second longitudinal portion 304. The third longitudinal portion 306 may have a third lateral width which varies along its length. The third lateral width may be equal to the first lateral width 320 of the first longitudinal portion 302 at an end of the third longitudinal portion 306 that is adjacent to the first longitudinal portion 302. The third lateral width may be equal to the second lateral width 322 of the second longitudinal portion 304 at an end of the third longitudinal portion 306 that is adjacent to the second longitudinal portion 304.

In other words, the third lateral width may decrease along the longitudinal axis 318 of the couch top 300 when moving towards the second longitudinal end 314 and away from the first longitudinal end 312. The rate of this decrease in width may be substantially constant. In other words, the first and second lateral sides 308, 310 of the couch top in the third longitudinal portion 306 may be straight lines. As further described herein, the first and second lateral sides 308 of the couch top in the third longitudinal portion 306 may be (diagonally) angled with respect to the longitudinal axis 318, e.g. may form an approximately 45° angle with the longitudinal axis 318.

The third longitudinal portion 306 may provide a gradual transition between the first longitudinal region 302 and the second longitudinal region 304. Considering the dose applied to a subject in a radiotherapy treatment, whether or not the radiotherapy beam passes through the couch top 300 or not can significantly affect the dose received by the subject. As such, when there is a sharp cut off between presence and absence of the couch top 300, small positional errors bridging the cut off can lead to large dosimetric errors. As further described herein, the gradual transition provided by the third longitudinal portion 306 can reduce the magnitude of such dosimetric errors resulting from small positional errors.

The first longitudinal portion 302 may be integrally formed with the second longitudinal portion 304. In examples in which the third longitudinal portion 306 is present, this may be achieved by the first longitudinal portion 302 being integrally formed with the third longitudinal portion 306 and the third longitudinal portion 306 being integrally formed with the second longitudinal portion 304, such that the first and second longitudinal portions 302, 304 are ultimately integrally formed. The different portions of the couch top 300 may be described as being integrally formed, being formed as one structure, being monolithic, and/or being formed without joints therebetween. As described herein, this can help prevent such joints distorting the images produced of the subject and/or can help prevent dosimetric discrepancies.

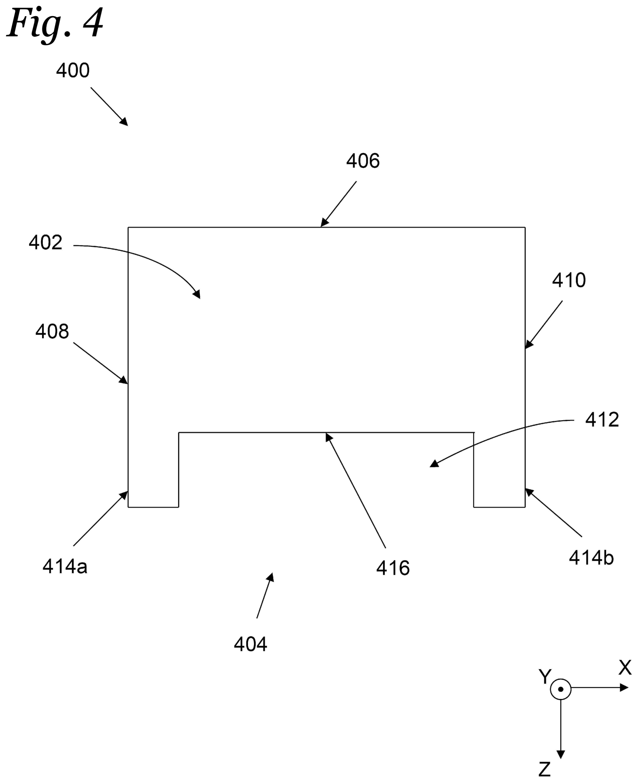

Figure 4 depicts an extension 400 according to the present disclosure. The extension 400 is suitable for coupling, connecting or fixing to the radiotherapy couch top 202, 300 as described herein. The extension 400 is configured to couple, connect or fix to the radiotherapy couch top 202, 300 as described herein. As used herein, the extension 400 may also be referred to as a cover board.

Figure 4 depicts a top-down view of the extension 400. The extension comprises a planar portion generally labelled as 402 in Figure 4. The planar portion 402 may also be described as a board or a flat surface. The planar portion 402 is configured to support at least part of a subject thereon, for example legs or arms of the subject. The planar portion 402 may be constructed from the same or similar materials to the couch top 202, 300. The planar portion 402 may be rigid enough to support the at least part of the subject without substantial bending of the planar portion 402. Since Figure 4 depicts a top-down view, an upper surface of the planar portion 402 is visible in Figure 4. The planar portion 402 comprises a lower surface opposite to the upper surface (and therefore not visible in Figure 4). A fixation mechanism (not shown in Figure 4) may be provided on the lower surface of the extension 400.

The extension 400 comprises a first longitudinal end 404 and a second longitudinal end 406. The extension 400 comprises a first lateral side 408 and a second lateral side 410. A width of the extension 400 between the first lateral side 408 and the second lateral side 410 may be equal to, or approximately equal to, the first lateral width 320 of the first longitudinal portion 302 of the radiotherapy couch top 300 (see Figure 3). As such, when the extension 400 is fixed to overlay the second longitudinal portion of the couch top 300, the combination of the extension 400 and the couch top 300 may be generally rectangular in form, and may have an approximately constant width along the longitudinal axis 318. When the extension 400 is fixed to overlay the couch top 300, the second longitudinal end 406 of the extension 400 may approximately overlay the second longitudinal end 314 of the couch top 300 (i.e. overlap with it along the Y-axis). When the extension 400 is fixed to overlay the couch top 300, the first longitudinal end 404 of the extension 400 may approximately overlay part or all of the third longitudinal portion 306 and may approximately overlay part of the first longitudinal portion 302.

The second longitudinal end 406 of the extension 400 may be generally linear/flat along the X-axis as depicted in Figure 4. The first longitudinal end 404 may also be generally linear/flat parallel to the second longitudinal end 406, but optionally may have the form depicted in Figure 4. As depicted in Figure 4, the first longitudinal end 404 may comprise a cutout 412. In other words, the first longitudinal end 404 may comprise first and second edge portions 414a, 414b which extend away from the second longitudinal end 406 (along the Z-axis) further than a central portion 416 extends away from the second longitudinal end 406 (along the Z-axis), thereby forming the concave/recess/cutout portion 412.

The cutout portion 412 may be provided to enable coupling of a positioning accessory in the cutout portion 412. In other words, the size and shape of the cutout portion 412 may be configured to receive the positioning accessory, which may be disposed on the couch top 300. The positioning accessory may comprise a knee block shaped to support the legs of a subject disposed on the couch top 300 under their knee joints. This may further increase the versatility of the patient support apparatus described herein, in particular in feet-first treatments.

One or more edges of the extension 400 may be chamfered, tapered or bevelled such that a thickness of the extension 400 (in the Y-direction) decreases gradually at its edges. For example, the first longitudinal end 404 (e.g. any one or more of the central portion 416 and the first and second edge portions 414a, 414b) of the extension may gradually decrease in thickness when moving towards the positive Z-direction as depicted in Figure 4. This gradual tapering of the thickness avoids sharp discontinuities in the material present in the treatment/imaging field, and therefore avoids sharp discontinuities in the amount these beams will be attenuated, which can increase the accuracy of the radiotherapy and imaging performed. In addition, comfort of the subject may also be increased, which is of particular importance for ensuring that the subject remains still during treatment.

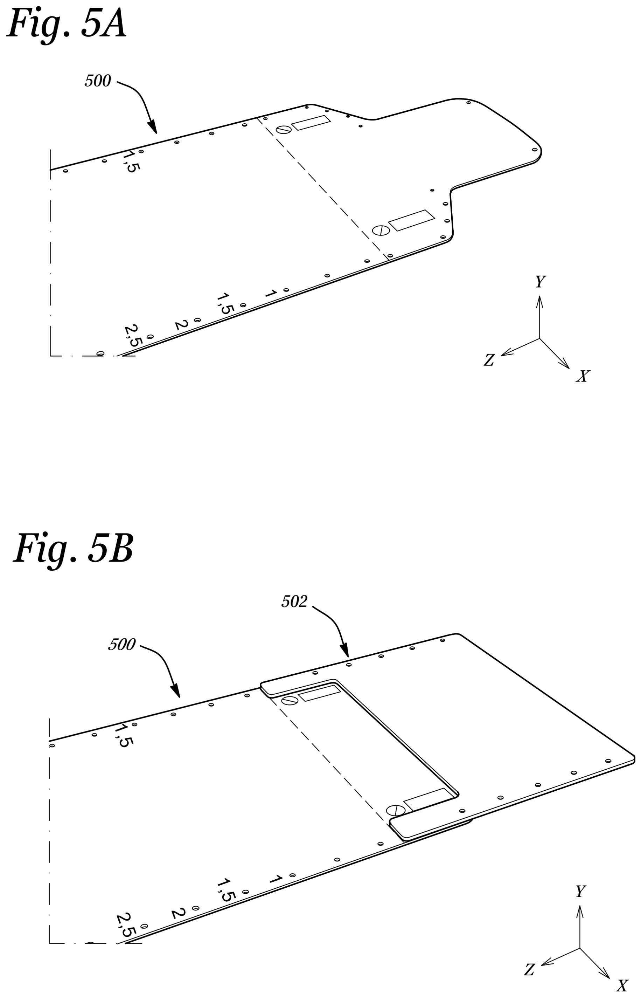

Figures 5a and 5b depict a couch top 500 and extension 502 according to the present disclosure. The couch top 500 and the extension 502 may correspond to and/or comprise corresponding features to any of the couch tops and extensions described herein. Figure 5a depicts the couch top 500 without the extension 502 present. In Figure 5b, the extension 502 has been fixed to the couch top 500 so as to overlay and be supported on part of the couch top 500. By way of example, the couch top 500 without the extension 502 may be used for head-first treatments, while the couch top 500 with the extension 502 fixed thereto may be used for feet-first treatments.

As described herein, the extension comprises a fixation mechanism, which may be on a lower surface of the planar portion of the extension. The fixation mechanism is configured to fix the extension onto the couch top such that it overlays at least part of the couch top. The couch top may comprise a second fixation mechanism configured to couple/fix to the fixation mechanism of the extension. The fixation mechanisms may respectively be integral with or permanently fixed to the extension/couch top of which they are a part, avoiding the presence of loose components.

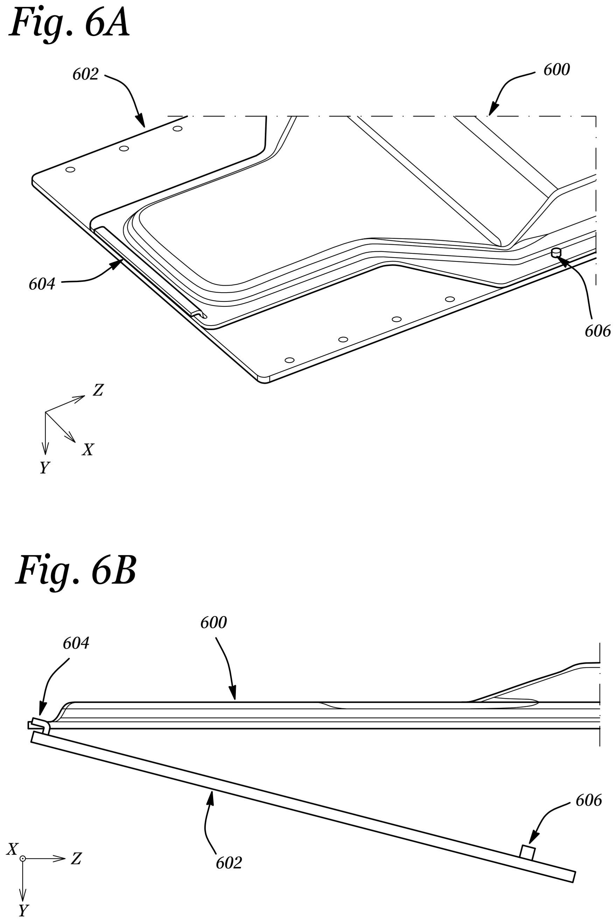

Any suitable form of fixation mechanism may be used. Certain advantageous fixation mechanisms are described below with reference to Figures 6a-b, 7 and 8a-b by way of non-limiting example. Multiple of the fixation mechanisms may be used in combination. The fixation mechanism, in combination with overlaying of the extension onto an upper surface of the couch top, enable the couch top to be securely held in the desired position.

Figures 6a and 6b depict an example of fixation of an extension 602 to a couch top 600 according to the present disclosure. The couch top 600 and the extension 602 may correspond to and/or comprise corresponding features to any of the couch tops and extensions described herein. Figure 6a depicts an underside of the extension 602 fixed to the couch top 600. Figure 6b depicts a side-on view of the extension 602 in the process of being fixed to the couch top 600, and is also depicted 'upside-down' (relative to Figures 1 and 2).

As depicted in Figures 6a and 6b, the fixation mechanism of the extension 602 may comprise a linear protrusion 604. The linear protrusion 604 may be described as a hinge or a lip which extends partially along a width of the extension 602 (along the X-axis). The linear protrusion 604 is configured to fit into a linear slit in the couch top 600 such that the extension 602 is rotatable with respect to the couch top 600 about the connection/hinge formed by the linear protrusion 604 and the linear slit in combination. The planar portion of the extension 602 may generally be disposed in the X-Z plane, and the linear protrusion may comprise a first portion extending from the planar portion parallel to the Y-axis and a second portion extending from the first portion parallel to the Z-axis, enabling the first and second portions to hook through and around the linear slit.

The length of the linear protrusion 604 along the X-axis may correspond to the length of the linear slit along the X-axis. The linear protrusion 604 and the linear slit may provide further support to the extension 602 on the couch top 600 to prevent flipping over of the extension 602 if a load is applied to a lateral side of the extension 602.

The linear protrusion 604, and the linear slit, may be disposed adjacent to the second longitudinal 406 (see Figure 4) of the extension 602/the second longitudinal end 314 (see Figure 3) of the couch top 600. This advantageously means that the fixation mechanism is at the extreme end of the couch top 600 and extension 602 such that it does not intrude into the treatment and imaging zones.

As depicted in Figures 6a and 6b, the fixation mechanism of the extension 602 may comprise one or more pins 606. The extension 602 may comprise first and second pins 606 on respective first and second edge portions 414a, 414b (see Figure 4) of the extension 602. Each of the pins 606 may be configured to fit within a corresponding hole of the couch top 600. The pins 606 and the holes may be dimensioned such that there is an interference fit between these features, such that friction between these components contributes to keeping the extension 602 in position. Since the pins 606 and the holes are at the lateral edges of the extension 602/couch top 600, rather than in a lateral centre thereof, they may also be kept from intruding into the treatment and imaging zones for many treatment/imaging scenarios.

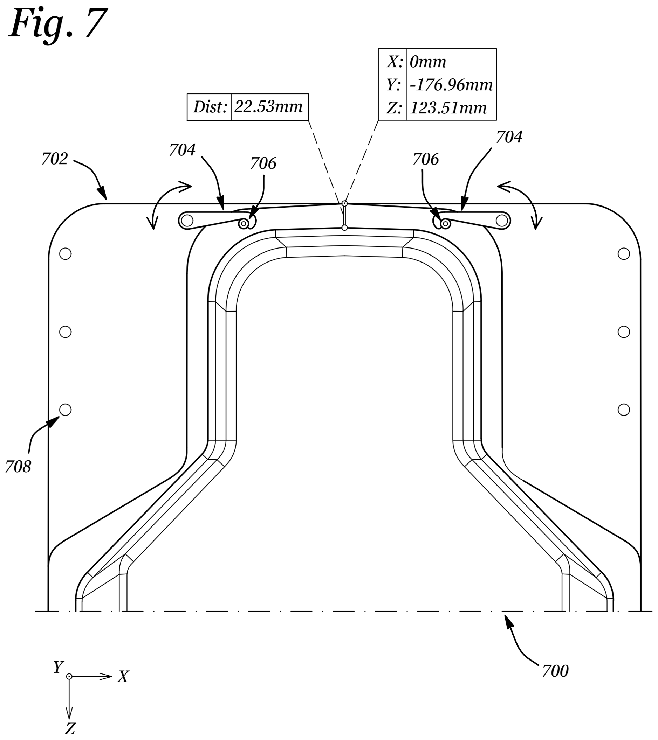

Figure 7 depicts a further example of fixation of an extension 702 to a couch top 700 according to the present disclosure. The couch top 700 and the extension 702 may correspond to and/or comprise corresponding features to any of the couch tops and extensions described herein. Figure 7 depicts an underside of the extension 702 fixed to the couch top 700.

As depicted in Figure 7, the extension 702 may comprise one or more hooks or latches 704. The hooks 704 may be rotatable about an axis parallel to the Y-axis (i.e. rotatable in the X-Z plane). The one or more hooks 704 may be disposed at the second longitudinal end 406 (see Figure 4) of the extension 702, such that they are kept from interfering with treatment and/or imaging zones. Each of the hooks 704 may be configured to couple to a respective pin 706 extending from the lower surface of the couch top 700 parallel to the Y-axis. Each hook 704 may be configured to at least partially wrap around the respective pin 706 so as to contribute to fixing/locking the extension 702 to the couch top 700.

As depicted in Figure 7, the extension 702 may comprise indexing holes 708. Any of the extensions described herein may comprise such indexing holes. Any of the couch tops described herein may also comprise similar indexing holes, which may be positioned to overlap with the indexing holes 708 of the extension (along the Y-axis). The indexing holes may contribute to positioning of the extension in the correct location relative to the couch top and/or may enable correct positioning of radiotherapy accessories to the extension and/or to the couch top.

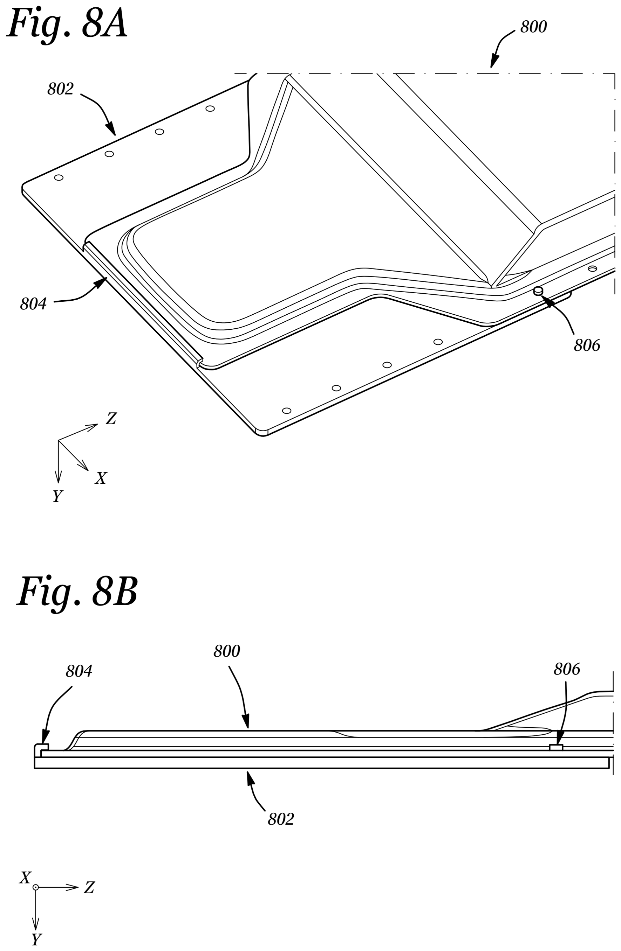

Figures 8a and 8b depict a further example of fixation of an extension 802 to a couch top 800 according to the present disclosure. The couch top 800 and the extension 802 may correspond to and/or comprise corresponding features to any of the couch tops and extensions described herein. Figure 8a depicts an underside of the extension 802 fixed to the couch top 800. Figure 8b depicts a side-on view of the extension 802 fixed to the couch top 800, and is also depicted 'upside-down' (relative to Figures 1 and 2).

As depicted in Figures 8a and 8b, the fixation mechanism of the extension 802 may comprise a U-shaped edge or lip 804. The extension 802 may generally be disposed in the X-Z plane, and the U-shaped edge 804 may curve downward along the Y-axis (i.e. towards the floor 206 of the room housing the radiotherapy device when in use). The U-shaped edge 804 may be disposed at the extreme second longitudinal end 406 (see Figure 4) of the extension 802, which may keep it from interfering with treatment and/or imaging zones. The U-shaped edge 804 may be generally linear in form along at least part of the width of the extension 802 (i.e. parallel to the X-axis). The U-shaped edge 804 may be dimensioned and configured to hook or curve over the second longitudinal end 314 (see Figure 3) of the couch top 800 so as to contribute to securely fixing the extension 802 to the couch top 800 and increasing the stability thereof.

As depicted in Figures 8a and 8b, the extension 802 may comprise one or more pins 806, which may correspond to the one or more pins 606 described in relation to Figures 6a and 6b. As also described for Figures 6a and 6b, the couch top 800 may comprise one or more holes corresponding respectively to the one or more pins 806 and configured to received them.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. An extension for increasing a width of at least part of a radiotherapy couch top, the extension comprising:
a planar portion configured to support part of a subject thereon; and
a fixation mechanism configured to fix the extension onto the couch top such that the planar portion overlays at least part of the couch top.

2. The extension according to claim 1, wherein the fixation mechanism is on a lower surface of the planar portion.

3. The extension according to claim 1 or claim 2, wherein the fixation mechanism comprises a linear protrusion for receiving into and rotating with respect to a linear slot of the radiotherapy couch top.

4. The extension according to any preceding claim, wherein the fixation mechanism comprises a pin for receiving into a hole of the radiotherapy couch top.

5. The extension according to any preceding claim, wherein the fixation mechanism comprises a rotatable hook for coupling to a pin of the radiotherapy couch top.

6. The extension according to any preceding claim, wherein the fixation mechanism comprises a U-shaped edge configured to hook over an edge of the radiotherapy couch top.

7. The extension according to any preceding claim, wherein the planar portion comprises a first longitudinal end and a second longitudinal end, wherein at least a first part of the fixation mechanism is disposed adjacent to the second longitudinal end of the planar portion,
optionally wherein at least one of:
A. the extension comprises a cutout at the first longitudinal end thereof configured to receive a positioning accessory;
B. the first longitudinal end of the planar portion comprises a chamfered edge;
and/or
C. the planar portion comprises a first lateral edge and a second lateral edge, wherein at least a second part of the fixation mechanism is disposed adjacent to the first lateral edge and/or the second lateral edge.

8. The extension according to claim 7, wherein no part of the fixation mechanism is disposed in a laterally central region of the planar portion adjacent to the first longitudinal end of the planar portion.

9. The extension according to any preceding claim, wherein the extension is configured to be selectively connectable to and separable from the radiotherapy couch top.

10. A patient support apparatus comprising:
a radiotherapy couch top; and
the extension of any preceding claim for fixing to the radiotherapy couch top using the fixation mechanism.

11. The patient support apparatus of claim 10, wherein the radiotherapy couch top comprises:
a first longitudinal portion with a first lateral width; and
a second longitudinal portion with a second lateral width, the second lateral width being smaller than the first lateral width,
optionally wherein the fixation mechanism is configured to fix the extension to the second longitudinal portion of the radiotherapy couch top such that it overlays the second longitudinal portion of the radiotherapy couch top,
further optionally wherein a lateral width of the extension is approximately equal to the first lateral width of the first longitudinal portion of the radiotherapy couch top.

12. The patient support apparatus of claim 10 or claim 11, wherein the combination of the radiotherapy couch top and the extension when fixed together are generally rectangular in plan view.

13. The patient support apparatus of any of claims 10 to 12, wherein the radiotherapy couch top comprises a second fixation mechanism configured to couple to the fixation mechanism of the extension,
optionally wherein at least one of:
A. the first longitudinal portion of the radiotherapy couch top is configured to support a torso of the subject;
B. the second longitudinal portion of the radiotherapy couch top is configured to support a head of the subject;
C. the extension is configured to support legs of the subject during feet-first radiotherapy treatments; and/or
D. the first longitudinal portion of the radiotherapy couch top is integrally formed with the second longitudinal portion of the radiotherapy couch top.

14. The radiotherapy couch top according to any of claims 10-13, comprising a third longitudinal portion of the radiotherapy couch top longitudinally between the first longitudinal portion and the second longitudinal portion, wherein the first longitudinal portion, the second longitudinal portion and the third longitudinal portion are all integrally formed with each other,
optionally wherein the third longitudinal portion has a third lateral width which decreases from being equal to the first lateral width at an end of the third longitudinal portion which is adjacent to the first portion, to being equal to the second lateral width at an end of the third longitudinal portion which is adjacent to the second longitudinal portion, and wherein the extension is configured to overlay the second longitudinal portion and the third longitudinal portion.

15. A radiotherapy device comprising:
a rotatable gantry;
a radiation source;
an imaging apparatus; and
the patient support apparatus of any of claims 10-14.
